# EUROPEAN PATENT APPLICATION

(11) **EP 0 945 107 A2**
(43) Date of publication of application: **29.09.1999**
(21) Application number: 99300469.6
(22) Date of filing: 22.01.1999
(51) Int. Cl.: A61F 2/06

(54) **Helical stent**

(30) Priority: 23.01.1998 US 72258; 29.01.1998 US 73035 P; 06.05.1998 US 84436 P
(71) Applicant: Arterial Vascular Engineering, Inc., Santa Rosa, California 95403 (US)
(72) Inventor: McGuiness, Colm, Galway (IR)
(74) Representative: Bayliss, Geoffrey Cyril

(57) **Abstract**

An expandable, high scaffold intraluminal stent (20) is provided having a plurality of undulations (28) disposed in a helical geometry about the surface (33) of the stent (20).

## Description

This application relates to the field of medical devices, and more particularly to vascular prosthesis for supporting a vessel wall.

Atherosclerosis is a condition that affects many individuals. Untreated, atherosclerosis may lead to severe consequences, including heart damage, heart attack and death. Known treatments for atherosclerosis have had limited success; for example, transluminal balloon angioplasty, wherein a balloon is inserted via a catheter into the artery of the patient and expanded, thus expanding the partially closed artery to a more open state, is a well-known treatment for atherosclerosis, but long-term benefits of angioplasty are limited by the problems of occlusion and restenosis, which results in re-closure of the artery.

A variety of intravascular stents and prostheses have been developed to support diseased arteries and thereby inhibit arterial closure after angioplasty. In particular, expandable intraluminal stents have been developed in which a catheter is used to implant a stent into the artery of the patient in a minimally invasive manner.

Expandable vascular stents are typically substantially cylindrical in shape and typically have a longitudinal lumen running through the center of the stent, which permits blood to flow through the artery when the stent is disposed therein. Such stents typically have two configurations: a first configuration of narrow radius for minimally invasive delivery of the stent through the vascular system of the patient to the diseased location and a second configuration of wide radius for support of the artery at the diseased location.

Stents may be expanded by a variety of mechanisms, depending on the geometry of the stent. Some expandable stents are constructed to have a spring-like resistance to compression, Such stents can be compressed during delivery and then permitted to expand when located in the diseased artery. Other stents are constructed of shape memory alloys, such as nitinol. Shape memory alloy stents can be compressed by cooling prior to or during delivery and are then caused to expand to a predetermined configuration when disposed in the diseased location by exposure to body temperature. Other stents are wound into helical spirals, so that rotation of the ends of the stent can cause the stent to expand or contract. Helical stents are typically subject to the drawback that the end of the stent is not square; that is, the end of the substantially cylindrical tube that defines the stent is not perpendicular to the longitudinal axis of the stent; instead, the end is at the angle of the spiral winding that makes up the stent.

A number of intravascular stents are balloon expandable. In balloon expandable stents, a balloon is typically disposed in the lumen of the stent when the stent is in a narrow radius configuration. The stent and balloon are disposed on a catheter, which is inserted through the vascular system to the diseased location. Inflation of the balloon causes the stent to be expanded to a wide radius configuration, which provides support to the arterial wall.

Stents present a number of difficulties for the surgeon such as insertion. A stent is typically inserted in the femoral artery and delivered via a catheter through the arterial system into the location of an arterial lesion; often near the heart. Because of the narrow twisting nature of the arterial system, it is desirable to have a stent be small enough to be inserted easily. A second difficulty with a stent is coverage; that is, a stent needs to be large enough to cover a lesion, and it needs to be of a shape that is suitable for covering the lesion, Moreover, the stent needs to provide a wall of sufficiently dense cross-section to prevent tissue from herniating or hanging through the wall of the stent, which can lead to thrombosis. Another difficulty is with location; that is, the stent needs to be desired so that it can be accurately located in the artery to cover the lesion.

Expandable stents are designed with a range of geometrics, materials and constructions. The expandable nature of such stents permits the stents to be delivered in a small configuration during insertion, then expanded to support the arterial wall.

An example of a balloon-expandable stent is a wire mesh tube. In such stents, intersecting wire members are secured at their intersections by welding, soldering or gluing, forming a wire mesh, diamond-like pattern. The diamond pattern is but one of a variety of known stent geometrics for stents of this type, but it is typical in that most such stents have some openings, or cells, between the members that make up the stent wall. The cells are desirable to permit contact between the vascular wall and the blood flowing through the artery. Some stents have a very tight mesh, resulting in many small cells. Other stents are relatively open, with a small number of larger cells. Thus, stent geometry in part determines the performance characteristics of the stent. As another example of the effect of geometry, the diamond pattern permits expansion and provides some resistance to radial collapse, but it permits little flexibility. Other stents provide relatively little support between wall members, so that they are quite flexible, but such stents may provide relatively weak wall support.

A particular problem posed by some stent geometries is a tendency toward longitudinal collapse or shrinkage upon expansion. Many stent geometries fail to provide longitudinal support during or after expansion. When exposed to longitudinal forces, which can occur during expansion or during the life of the stent in the intravascular environment, such stents may become shorter, providing less wall coverage and thus less protection from restenosis. Length contraction, also makes stents difficult to locate properly in the artery. A surgeon, typically using a fluoroscope,_{,} must locate the stent during a brief period of fluoroscopy and place it in the artery at the location of the lesion prior to expansion. If the stent shrinks in length during expansion, it may not cover the lesion completely.

Other stent geometries provide longitudinal support, but are inherently subject to some shrinkage due to their geometry. An example is the diamond pattern stent. The diamond pattern permits radial expansion, as the top and bottom apices of a particular diamond-shaped cell become more distant, but the resulting reduction of the distance between the side apices of a given cell results in an overall reduction in the length of the stent. Thus, a need exists for stent cell geometries that permit radial expansion while substantially maintaining length.

Stent geometries have been developed that are intended to permit expansion without causing a substantial inherent contraction in length. In particular, a wire-type, balloon-expandable stent is disclosed in U.S. Pat. No. 4,886,062 issued to Wiktor. The stent disclosed in Wiktor is constructed from a deformable wire bent into a zig-zag design and coiled into a helical spiral. The opening of the acute angles of the zig-zags during radial expansion compensates in part for any reduction in length caused by radial expansion. However, due to the nature of its construction, the Wiktor stent is subject to a number of drawbacks shared by most wire-type stents, as described below.

The selected method of stent construction greatly affects not only stent geometry but other important performance characteristics of a stent. Stents may be constructed by a variety of methods, resulting in very different types of stents. The majority of stents can be classified either as slotted tube stents or as wire stents. Slotted tube stents are typically formed by a negative process of removing material from a solid tube. Thus, the wall openings of a slotted tube stent are created by removal of material, rather than by construction or manipulation of wall members into the selected shape. Removal of the material may be accomplished by a variety of means, such as laser cutting or photochemical etching. An alternative method of constructing a tubular stent consists of cutting or etching a ribbon of material that is then rolled into a tube, with adjacent edges of the pattern being glued, soldered or welded together.

Wire stents are typically formed by bending or wrapping a wire into a desired configuration. Depending on the stent geometry, the wall members formed by shaping the wire may then be connected by various means, including welding, soldering, gluing, hooking, looping, or tying the members. In some wire stents, such as the Wiktor '062 stent, the wall members are not connected.

Slotted tube stents are typically quite different from wire stents. In such stents, a plurality of slots are formed, as by removing material through cutting, etching etc. from the wall of the tube. These slots permit radial expansion of the stent as opposed to wire-type stents where the wire is formed in a manner so as to permit radial expansion without appreciable foreshortening. For example, a tubular stent is typically stronger and substantially less deformable, once in the greater radially expanded position, than is a wire stent. A tubular stent typically has a relatively closed configuration, with many small cells, in contrast to a wire stent, which typically has an open configuration with a small number of larger cells. The deformability of most wire stents can be a significant drawback, because such stents provide less support to the diseased artery. Under strong forces, such as those encountered by the stent in the vascular system, such stents can partially radially collapse, reducing the blood flow through the diseased portion of the artery. Due to their flexibility, wire stents can also collapse in length, reducing stent coverage, unless separate support members, such as a longitudinal spine, are provided. The open construction of wire stents can also be a significant drawback. Large cells can permit plaque elements to extend through the cells into the interior of the stent. Plaque in the interior of the stent can cause a buildup of tissue or thrombosis.

Although tubular stents are strong, the more rigid, small-cell construction of tubular stents can also present drawbacks. At times it is desirable to have access to the interior of the stent through an opening other than the end, such as when the stent is positioned at a branch in the vascular system. The typically tight mesh of most tubular stents inhibits side branch access. Also, known geometries of tubular stents are subject to length contraction upon radial expansion, which reduces the area of support covered by the stent.

Known, stents do not solve all of the difficulties presented to the practitioner. It is very difficult to provide a wire stent of sufficient strength to prevent contraction under pressure and sufficient wall density to preclude herniation of tissue and thrombosis. However, known tubular stents, which provide strength and wall density, typically do not maintain length upon expansion. Accordingly, a need exists for a tubular stent that is constructed in a geometry that permits radial expansion without length contraction.

This application relates to a vascular prosthesis, and more particularly to an expandable stent comprising undulations disposed in a pattern about the circumference of the stent. The undulations are preferably in a helical pattern. The expandable stent may further include connecting rods connecting a plurality of the undulations and providing wall support while permitting radial expansion in the absence of significant longitudinal contraction.

According to one embodiment, the stent includes a plurality of undulations in a helical path about a longitudinal axis to define a surface, with the peaks of the undulations connected by connecting rods. In different embodiments, connecting rods are provided in S-, Z-, J-, U-, reverse-S, and other shapes, and connecting rods are connected either between all undulations or alternating undulations in successive bands of the stent.

In another embodiment the stent includes undulations disposed about a surface in substantially helical bands with connecting rods connecting the undulations at a plurality of peaks of the undulations. Furthermore, the connecting rods may include a plurality of curved portions and a plurality of substantially straight portions between such curved portions, wherein a first curved portion is oriented oppositely to a second curved portion. The connecting rods may also be substantially Z-shaped. The end bands of the undulations terminate the stent in a substantially square end wherein at least one of the end bands includes a marker identifying the end band. The term "Z-shaped," as used, encompasses any structure that includes sharp angles that are substantially oppositely oriented. In other embodiments, corresponding peaks of successive bands of the undulations are aligned substantially in parallel with the longitudinal axis.

In certain embodiments of the stents, the stent is provided with a square end; that is, the stent is provided with a pattern of undulations that terminate in a plane substantially perpendicular to the longitudinal axis of the stent. This is in contrast to a typical helical stent, in which a wound coil or helix results in the stent having an angled offset at the end, so that undulations or coils terminate in a place that is at an acute angle to the longitudinal axis of the stent. To accomplish the square end, undulations of one or both ends of the stent may include progressively different length undulations, so that the angle of the helical pattern of the stent is offset by the different length undulation. The progressively different length undulations may be used to square the end of any stent that otherwise has an angled end, including those having non-helical patterns. Thus, a square end, or an end in which undulations terminate in a plane perpendicular to the end of the stent, provides full circumferential coverage at the proximal and distal ends of the stent.

A method of stenting in a region of bifurcation, is also provided, which includes providing an expandable stent having an exterior wall including a helical pattern of undulations having peaks and connecting rods that connect the peaks and that define a space therebetween; positioning the stent in a first location; expanding the space defined by the connecting rods; and disposing a second stent through the space into a second location. In some embodiments, the connecting rods connect alternate peaks of the undulations. In certain embodiments, expanding the space is accomplished by inserting a balloon in the space and expanding the balloon.

Methods of making expandable prostheses are also disclosed and comprise providing a tube of biocompatible material; and removing a portion of such biocompatible material to leave undulations in a helical pattern about the tube. These methods can include processes that provide a cylindrical tube of biocompatible material and remove portions of the biocompatible material to leave undulations and connecting rods,where the undulations are disposed in a helical pattern about the cylindrical tube and the connecting rods connect the undulations. Further, the method can include leaving undulations that form a plurality of bands about the cylindrical tube, and providing an end band of undulations having progressively different length undulations to provide a square end of the stent. It should be understood that the term "removing" includes any possible method or device for removing material, such as etching, whether photochemically or otherwise, cutting, including laser cutting, slicing, punching, or drilling.

The invention may be further understood by reference to the following figures:
FIG. 1 illustrates one embodiment of a high scaffold stent;
FIG. 2 illustrates in greater detail the stent of FIG. 1;
FIG. 3 depicts the connecting rod pattern of an embodiment of the stent;
FIG. 4a depicts the pattern of an embodiment of the stent in which the end bands are wrapped so that they end in a square end;
FIG. 4b depicts another vice of the embodiment of FIG. 4a;
FIG. 5 depicts the pattern of an embodiment of the stent in which all of the undulations are lengthened;
FIG. 6 depicts the pattern of an embodiment of the stent in which each end band includes markers.
FIG. 7 illustrates the pattern of an embodiment of the stent in which connecting rods are placed between alternate peaks of the stent
FIG. 8 illustrates an embodiment of the stent in which a procedure of bifurcate stenting through side branch access is depicted
FIG. 9 depicts the pattern of an embodiment of the stent in which a band is separately cut or etched and then wrapped and joined at the peaks to form the stent.
FIG. 10 depicts the pattern of an embodiment of the stent in which the connecting rods are in an S shape.
FIG. 11 depicts the pattern of an embodiment of the stent in which the connecting rods are in an S shape, and alternate connecting rods are removed or not included.
FIG. 12 depicts the pattern of an embodiment of the stent in which the connecting rods are in a J shape.
FIG. 13 depicts the pattern of an embodiment of the stent in which the connecting rods are in a J shape, and alternate connecting rods are either removed or not provided.
FIG. 14 is a illustration of the stent in an expanded state.
FIG. 15 depicts the orientation of the undulation peaks in an embodiment of the present invention.
FIG. 16 depicts an embodiment, of the invention with a specially modified end module to square off the end of the stent.
FIG. 17 depicts the pattern of an embodiment of a stent of the present invention in which the connecting rods are in a Z shape, a special square end module is provided, and spaces for radiopaque markers are absent.
FIG. 18 depicts the pattern of an embodiment of the present invention in which the connecting rods are in a Z shape, and a special end module is provided to give the stent a substantially square end.
FIG. 19 depicts the pattern of an embodiment of the present invention in which the connecting rods are in a substantially Z shape, and special connecting members provide a substantially square end band for the stent.
FIG. 20 depicts the pattern of an embodiment of the present invention in which the connecting rods are in a Z shape, and a substantially square end band is provided which is connected to the other bands of the stent by a substantially Z-shaped connecting member.
FIG. 21 depicts the pattern of an embodiment of the present invention in which the connecting rods are in a Z shape, successive wraps of the stent are at an angle sufficient to provide space for two separate wrapping bands of the stent, and two bands are disposed in the pattern of the stent.
FIG. 22 depicts an embodiment of the present invention in which the connecting rods are in a substantially "reverse-S" shape, peaks of successive wraps are aligned perpendicular to the angle formed by the wraps, and the wraps are at an angle to provide space for three successive wraps about the stent.
FIG. 23 depicts an embodiment of the present invention in which two successive wraps are provided, peaks are angled perpendicular to the angle of the wraps, and U-shaped connecting rod are provided.
FIG. 24 depicts an embodiment of the present invention in which three successive wraps are provided connected by Z-shaped connecting rods, and in which special end modules are provided to give the stent a substantially square end.
FIG. 25 depicts an embodiment of the present invention in which radiopaque material is used for end bands of a stent.

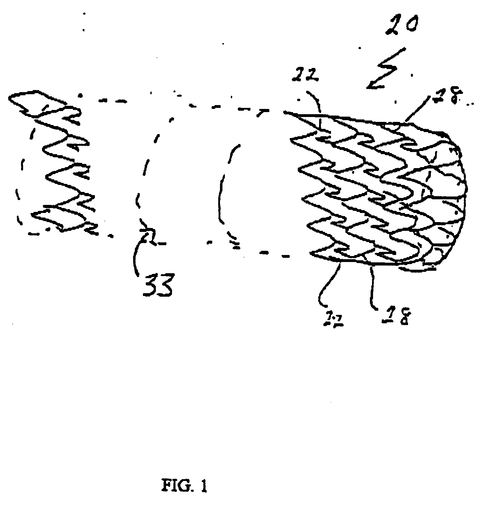

Referring to FIG. 1, in one embodiment, a high scaffold stent or prosthesis 20 is illustrated. The terms "stent" and "prosthesis" as used herein, can include any stent, graft, prosthesis, support or other structure for supporting a wall, such as an intravascular arterial wall, The stent depicted in FIG. 1, and the other stents depicted herein, are expandable. The term "expandable" encompasses any property, device or technique for permitting a stent to alter in radius, including balloon expansion or use of shape memory alloys.

The stent may include undulating wall members, or undulations 28, disposed about a surface 33. The term "undulation" can be understood to encompass any structure, such as a zig-zag, that is disposed along a line or course that proceeds in altering directions and should be understood to encompass both patterns that have sharp changes in direction, such as the letter "Z," and patrons that have smooth changes in direction, such as the letter "S," the letter "J," or a sinusoidal pattern.

The undulations 28 may be connected by connecting members, or connecting rods 22, of various designs. The term "connecting rod," as described herein, should be understood to refer to any connecting rod or member that is capable of attaching one or more structures, such as the undulations of the stents disclosed herein. In certain embodiments, the connecting rods of the tubular stents disclosed herein may include connecting rods that seamlessly connect the undulations. The term "seamless," as used herein, refers to a joint or connection that is made without the need for a separate weld, solder, or other attachment step.

The connecting rods 22 may include opposing curved portions, forming an S-like shape. The high scaffold stent may be provided in a number of different embodiments, reflecting different patterns of undulations 28 and connecting rods 22. In certain embodiments depicted below, material is removed from a tubular member, while other embodiments may be provided through other methods of stent manufacture, such as winding or bending a wire into the desired pattern. The disclosure should be understood to encompass any method of making a stent of the desired pattern, including laser cutting or etching from a tube, laser cutting or etching a band of' material that is then wrapped into a tube, or wrapping or bonding a wire into the desired pattern.

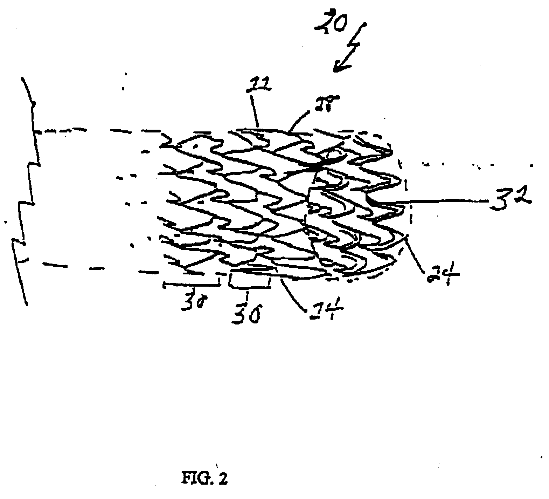

Referring to FIG. 2, in a first embodiment, the stent 20 includes a band 30 disposed substantially in a helical geometry about the circumference of an opening 32. The term "helical," as used herein, should be understood to include any helix or similar pattern that is disposed about the circumference of a cone, or similar structure at a substantially constant angle to a longitudinal axis of the structure. The term "band," as used herein, should be understood to encompass any substantially two-dimensional strip or area, including, for example, an area disposed about part of the circumference of a tube. The term "end band," as defined herein, should be understood to include a band of area disposed circumferentially about the end of the stent.

The stent illustrated in FIG. 1 includes connecting rods 22 of a reverse "S" shape, connecting each peak 24 of a undulation 28 to a corresponding peak 24 of a previous turn of the hand 30. The term "peak," as used herein in connection with the term "undulation", should be understood to encompass any structure that is located at a turn of a undulation, whether a sharp turn, or a smooth turn.

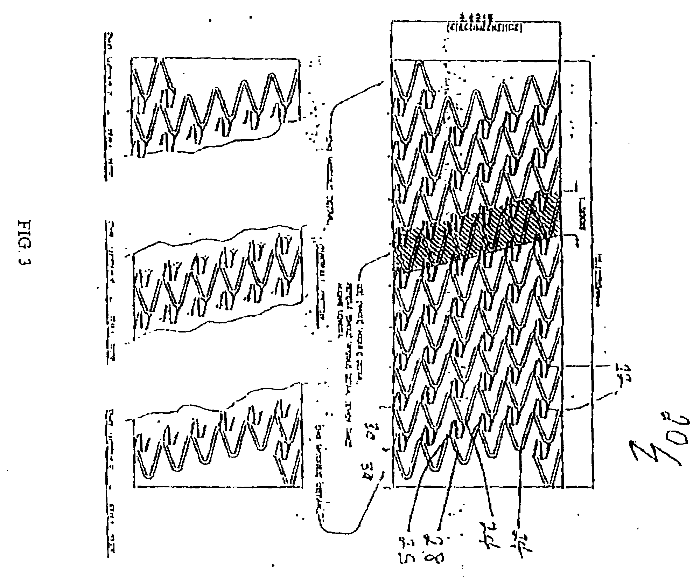

FIGs. 3 - 7 and FIGS 10-14 are two dimensional depictions of different patterns of the connecting rods 22 and undulations 28 of the stent 20. The patterns may also be created by other methods, such as photochemical etching, or by forming wires into the patterns depleted. It should be understood that all stents having the disclosed patterns or other patterns combining the elements disclosed in the figures are within the scope of the present disclosure.

Referring to FIG. 3, in an embodiment of the stent 20, connecting rods 22 are shown in detail between peaks 24 of undulations 28. The connecting rods 22 are in a reverse "S" configuration and are disposed at an angle between a peak 24 of a turn of the band 30 and a corresponding peak 25 of another turn of the band 30.

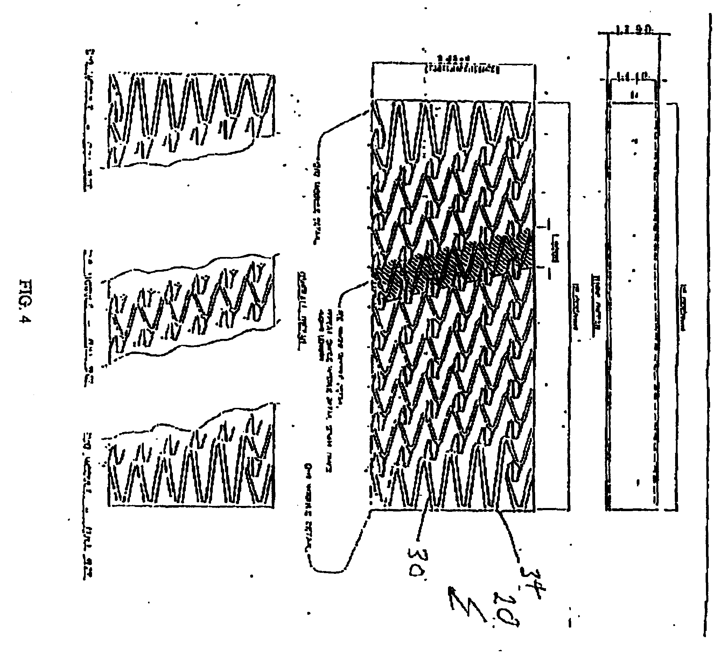

FIG. 4a depicts another embodiment of the stent 20, the turns of the band 30 are wrapped so that they end in a substantially square end 34 to the stent 20, and the turns of the band 30 near the ends of the stent 20 are of progressively increasing length, facilitating deployment in an artery.

Referring to FIG. 4a and FIG. 4b, as used herein, the terms "end band" and "end module" refer to an area of arbitrary size at one or more the ends of the stent 20. A stent 20 having an end turn of the band 30 or an end module that is substantially perpendicular to the longitudinal axis of the stent may be described as having a "substantially square end" and include full circumferential coverage at the proximal and distal ends of the stent. The embodiments depicted in FIGs. 4a and 4b tend to assist in allowing uniform opening of the stent 20 under pressure from a balloon, because the end turns of the band 30 are thicker at the points (the ends of the stent 20) at which the balloon pressure tends to be greatest during initial inflation, when portions of the balloon may extend beyond the end turn of the band 30 of the stent 20. The thicker end turn of the band 30 also increases radiopacity at the end of the stent 20.

Referring to FIG. 4b, in one embodiment, the end turn of the band 30 or end module terminates along or near a circle 35 that both is co-axial with the longitudinal axis 31 of the stent and that has a diameter 36 that is substantially perpendicular to the longitudinal axis 31 of the stent. Such a "square end" is thus the type of end one would obtain upon cutting a cylindrical stent at an angle perpendicular to the longitudinal axis of the cylinder, as opposed to an "offset end," or "angular end," which would result if the cylinder were cut at an angle substantially different from ninety degrees relative to the longitudinal axis of the stent A "square end" encompasses structures in which the peaks of undulations of the end band or end module of the stent terminate along or near such a circle. That is, a repeating pattern of undulations, in which certain undulation peaks terminate along the circle that is perpendicular to the longitudinal axis of the stent is encompassed by the term "square end" as used herein, even though the peaks only fill part of that circle. Similarly, an alternating pattern of long and short undulations about the circumference of the end of the stent, in which some undulation peaks are longer and others are shorter, but in which an arbitrary angular rotation of the stent does not substantially change the coverage of the stent is described herein as a "square end."

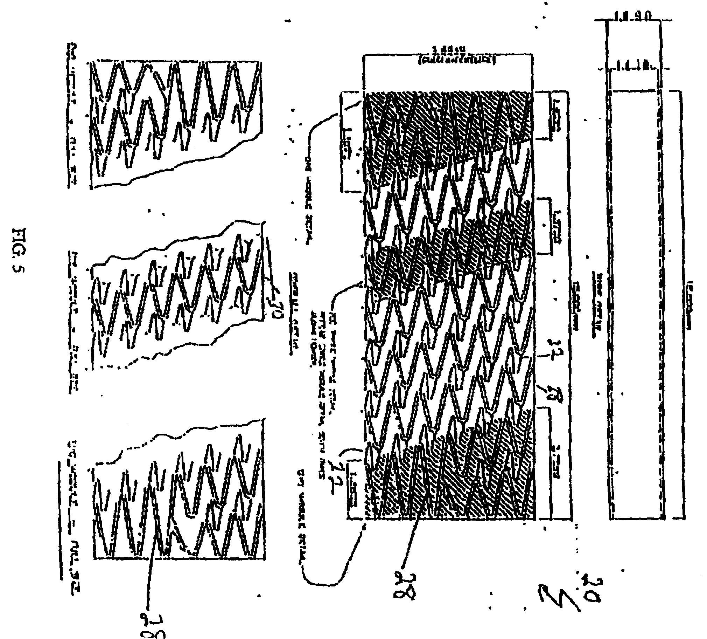

Referring to FIG. 5, in another embodiment, the undulations 28 are lengthened to facilitate expansion to large diameters. It should be recognized that larger diameters are also established by providing additional undulations 28 in each turn of a band 30 in a given pattern. In one such embodiment, a diameter of approximately five millimeters is possible for a coronary stent, or a diameter of approximately 5-12 millimeters for a peripheral stent.

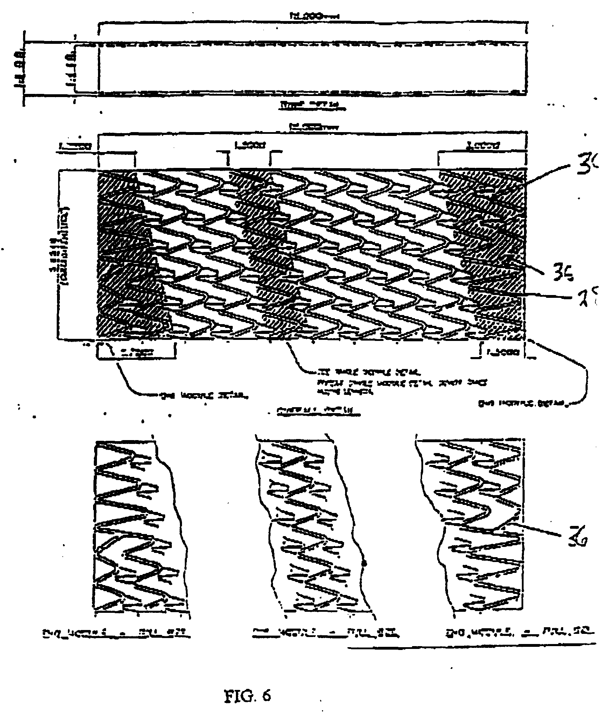

Referring to FIG. 6, in another embodiment, a square end band 30 is provided, and end turn of the band 30 includes markers 36 to permit identification of the end turn of the band 30. The radiopaque markers 36 are placed into the insert spaces cut or etched into one or more undulations 28 of the band 30 end turn and depicted in FIG. 6. Gold, platinum, tantalum or other radiopaque materials can be inserted into the spaces that are laser cut into the stent pattern. The different configuration of the end turn of the band 30 permits insertion of radiopaque markers to assist viewing of the stent 20 *in vivo*.

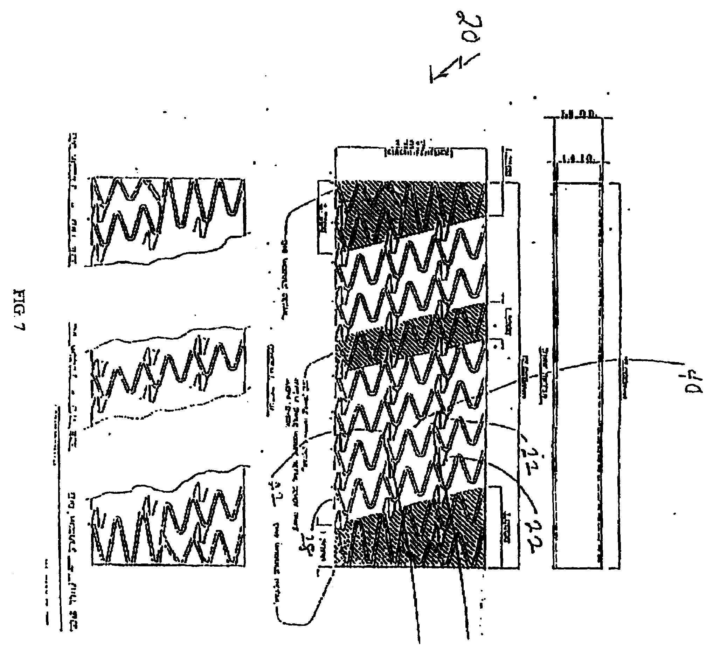

Referring to FIG. 7, in another embodiment, connecting rods 22 are placed between some, but not all adjacent peaks 24 of adjacent turns of a band 30 of the stent 20. This may be accomplished either by laser cutting or etching the tubular stent into this configuration, or by removing some connecting rods 22 from a stent 30 as disclosed in the first embodiment disclosed above. This embodiment permits side branch access into the stent 20, as may be needed when the stent 20 is placed at a branch of two arteries. This embodiment also permits additional flexibility in the stent 20. To further assist in the placement of the stent during the stenting of a bifurcated region, radiopaque markers may be inserted in the connecting rods adjacent the removed connectors.

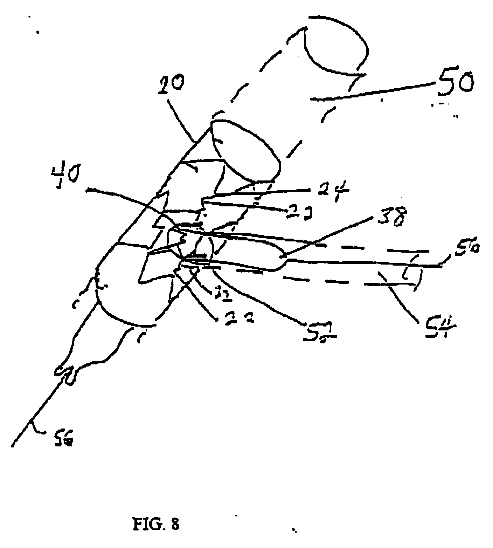

Referring to FIGs. 7 and 8, an embodiment of the stent 20, as well as other embodiments that have connecting rods 22 between only some peaks 24 of an adjacent turn of a band 30, can be used in a procedure of bifurcate stenting in which the stent 20 is remodeled after deployment by inflating a balloon 38 that is disposed in the space 40 where the connecting rod 22 is omitted or removed in this design.

Referring to FIG. 8, in a first step or a procedure of bifurcate stenting, the stent is deployed in a main vessel 50, with the space 40 disposed over an opening 52 to a side branch vessel 54. Next, a guide wire 56 is trained through the space 40 into the side branch vessel 54. Next, a balloon, such as a 3.0 mm balloon, is deployed in the space 40 of the stent 20 over the wire 56, so that the balloon is centered across the wall of the side branch vessel 54. Once the balloon 38 is in position, it is inflated. In addition, in some instances, the connecting rods 22 of the nearest peaks 24 are bent into the side branch vessel 54, providing some support to the side branch vessel 54. The balloon 38 and wire 56 may then be removed, leaving the stent in this configuration. The side branch vessel 54 then may be accessed through the space 40 and stented in a conventional fashion.

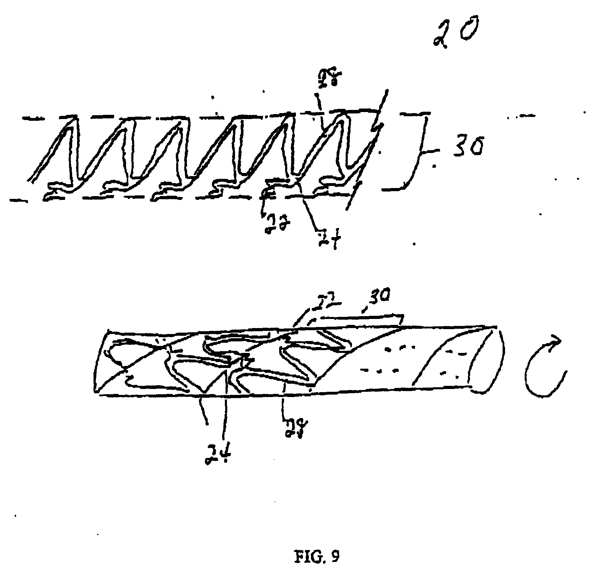

Referring to FIG. 9, in another embodiment of stent 20, a band 30 can be separately laser cut, photochemically etched or formed from a wire and then wrapped and joined at the peaks 24 by connecting rods 22 to form the stent 20. Joining may be by conventional means, such as gluing, welding, soldering or other methods. A stent 20 of any of the configurations disclosed here may be manufactured with this process.

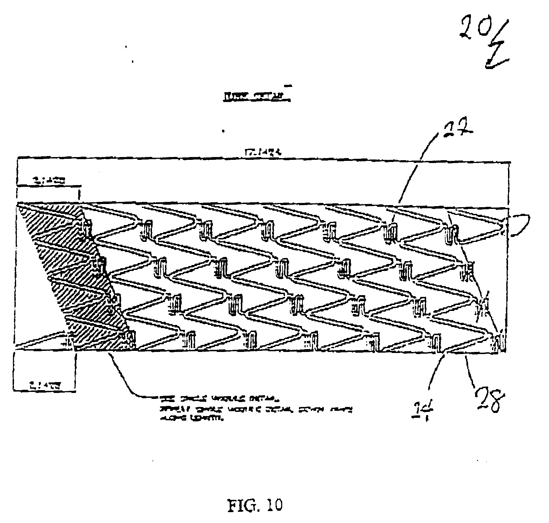

Referring to FIG. 10, in another embodiment, the connecting rods 22 are in an S shape.

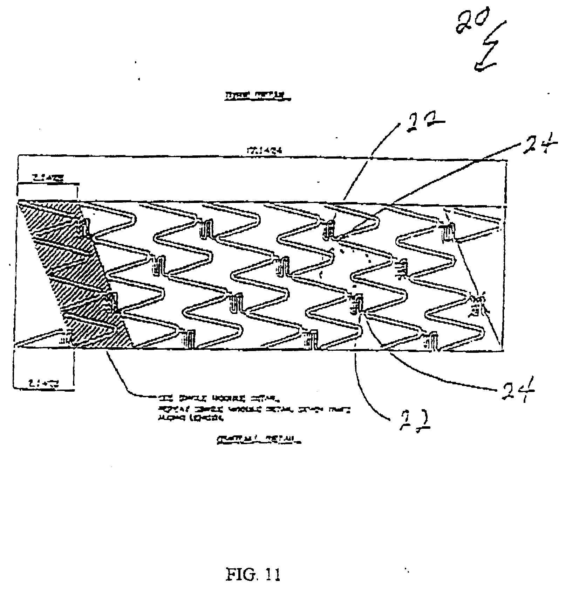

Referring to FIG. 11, in another embodiment, the connecting rods 22 are in an S shape, and alternate connecting rods 22 are removed or not included.

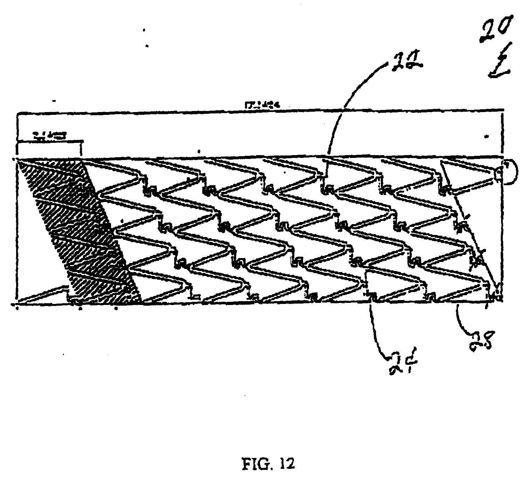

Referring to FIG. 12, in another embodiment, connecting rods 22 are in a "J" shape,

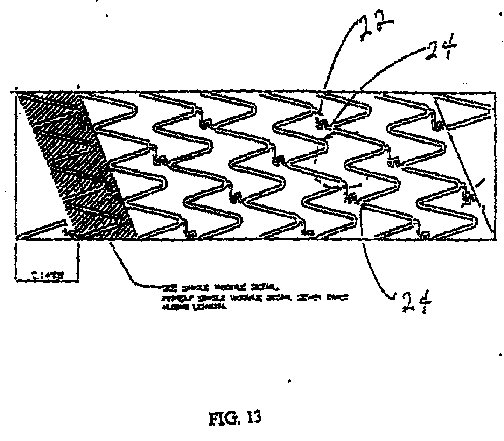

Referring to FIG. 13, in another embodiment, connecting rods 22 are in a "J" shape, and alternate connecting rods 22 are either removed or not provided.

The high scaffold stent may be manufactured by laser cutting a pattern into a thin tube of material, which may be metal, such as stainless steel, or another biocompatible material. The term "biocompatible material," as used herein, should be understood to encompass any material that can be disposed in the human body without significant adverse effects, including stainless steel, gold, tantalum, ceramics, and other materials. These terms should be understood to encompass materials that are covered, treated, painted, dipped, plated, irradiated, or coated to provide biocompatibility. An alternative method involves photochemical etching a metallic ribbon, which is then coiled into a tube, with adjacent wraps being joined by laser welding the end of one connecting rod tip to the appropriate peak of the adjacent undulation.

Any of the connecting rod patterns disclosed in the above-referenced figures can be manufactured by laser cutting a tube of stent material into the disclosed pattern. The various geometries result in stents that are strong and resistant to radial compression. The cells of the stents of the present invention are small, inhibiting the herniation of arterial material into the interior of the stent, thus reducing the likelihood of thrombosis. The end cells of certain embodiments of the present invention avoid the problem of wire stents that loose ends must be terminated to prevent damage from sharp ends.

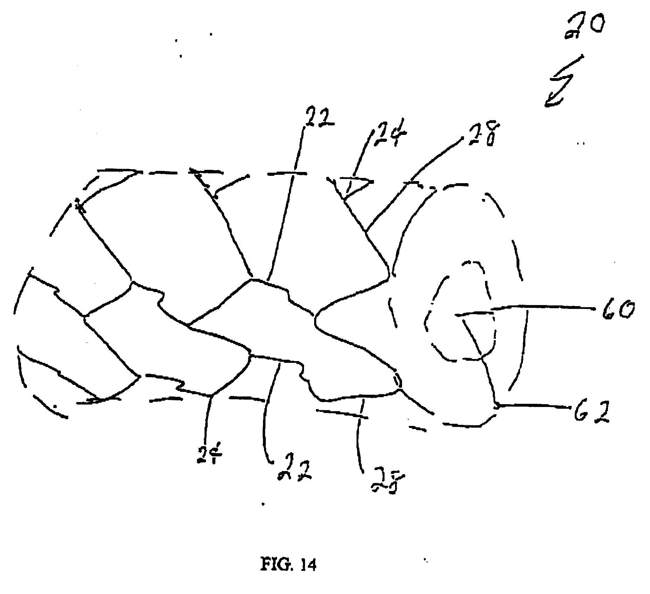

In each of the embodiments, the radial expansion of the stent 20 is permitted by the opening of the curves of the peaks and troughs of the undulations 28. FIG. 14 depicts an embodiment of the stent 20 in an expanded state. The stent 20 is expanded from an initial diameter 60 to a larger diameter 62. The opening of the curves of the connecting rods 22 substantially, although not perfectly, balances any foreshortening that would be normally caused by the radial expansion of the stent via the opening of the undulations 28. Thus, the stent 20 remains substantially the same in length upon any changes in radius. The connecting rod designs also provide additional wall support between the adjacent undulations 28, as well as additional radial strength. The every peak connecting rod design disclosed in FIG. 3 provides additional wall strength and helps prevent material from hanging through the wall of the stent and into the artery. Such material could result in thrombosis. The alternate peak design permits side channel access to the interior of the stent, as described above.

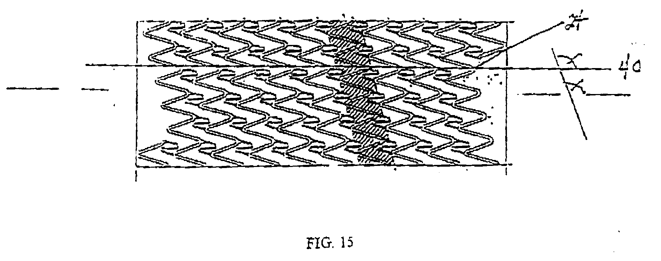

Referring to FIG. 15, the connecting rods 22 of an embodiment of the present invention provide another advantage in that the peaks 24 are aligned substantially in parallel with the longitudinal axis of the stent 20. That is, a line 40 bisecting a given peak is substantially parallel to the longitudinal axis 42 of the cylindrical lumen of the stent 20. This alignment of the peaks may provide strength to the stent 20 in the longitudinal direction in particular, the stent 20 may be more resistant to compression in the longitudinal direction than is a stent of similar material and manufacture that has peaks that are at an acute angle to the longitudinal axis. This orientation of peaks also imparts more uniform expansion and crimping when the stent is radially altered.

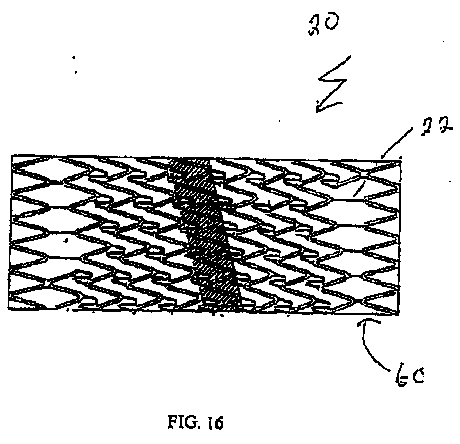

Referring to FIG. 16, in another embodiment of the stent 20, a specially designed end module 60 is provided to square the end of the stent 20. The connecting rods 22 of the end module 60 of this embodiment of the stent 20 are substantially straight. This arrangement of connecting rods 22 contributes to increased crimpability of the end module 60, relative to end modules of other embodiments of the stent 20.

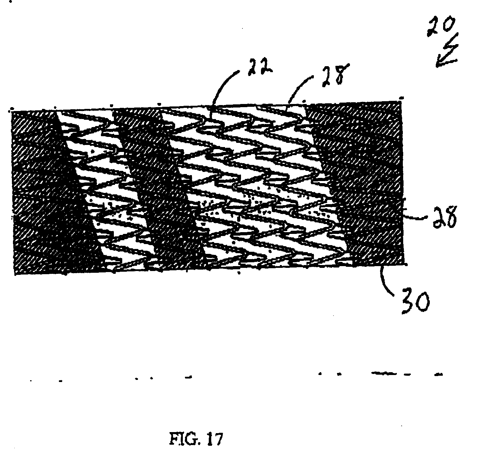

Referring to FIG. 17, in another embodiment of the present invention, a square end module is provided, in which the end turn of the band 30 is similar to the end band 30 depicted in the embodiment of FIG. 6. In the embodiment of FIG. 17, there are no spaces for radiopaque markers as there were in the embodiment of FIG. 6. The end of the stent 20 in the embodiment of FIG. 17 can be identified by the different configuration of the undulation 28 of the end module, which results in a substantially square end of the stent 20. In particular, about the circumference of the end module or end turn of the band 30 of the stent 20, the end module includes undulations 28 of progressively different lengths, so that each undulation 28 makes up the distance between a peak of a previous turn of the band 30 and a line that is perpendicular to the longitudinal axis of the stent 20. In other words, the progressively different length undulations 28 make up for the offset of the tubular shape of the stent 20 that results when undulations are disposed in a substantially helical spiral geometry about the circumference of the stent 20. This embodiment also includes mid-strut termination of the end turn of the band 30.

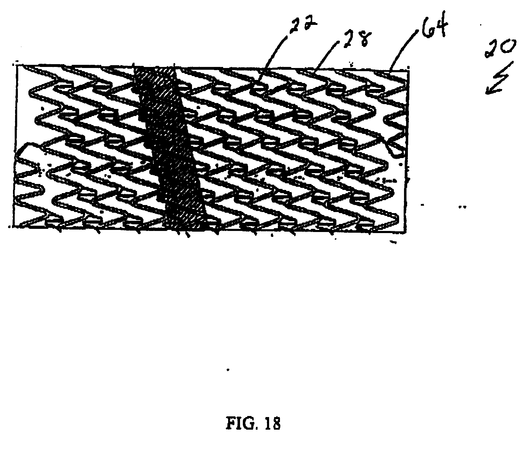

In the embodiment depicted in FIG. 18, the end module provides a termination joint of the helical undulations. As used herein, "termination joint" should be understood to encompass any structure for the joining, connection, or attachment of a band, undulation, connecting rod, end module, or end band, including a joint at an end band or end module of the stent. It should be understood that the term "termination joint" may encompass a seamless interface or connection between structures that were at no time discrete structures. For example, a connecting rod may connect two undulations, with all three elements, including the connection, being created by laser cutting away all material other than the material that forms the joint. The termination joint depicted in FIG. 18 may have progressively different length undulations.

Referring to FIG. 18, in an embodiment of the present invention, the final turn of the band 30 of the undulations 28 is a partial turn 64 that connects back to a peak of a undulation 28 of a previous turn of the band 30. The partial turn 64 provides the stent 20 with a substantially square end, rather than the offset end of an undulation pattern formed by a single turn of the band 30.

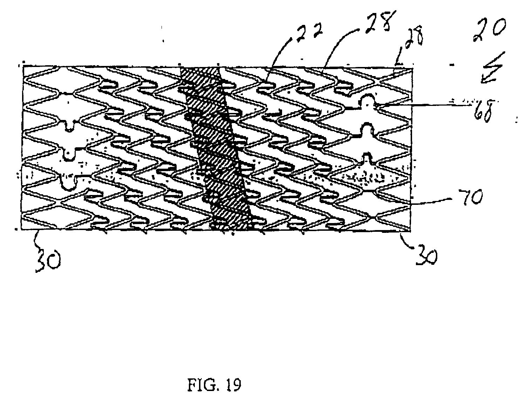

Referring to FIG. 19, in another embodiment of the present invention, the turn of the band 30 that ends the stent 20 is shaped so that the peaks of the undulations 28 of such end turn of the band 30 are aligned along a line that is perpendicular to the longitudinal axis 32 of the stent 20, giving the stent a substantially square end. The end turn of the band 30 is connected to the undulation pattern of the stent 20 by connecting members 68, 70. Certain connecting members 68 are substantially "U" shaped, while other connecting members 70 are substantially straight. The connecting members 68, 70 offset the end turn of the band 30 from the angled undulations 28 of the stent 20 to a varying degree sufficient to permit the end band 30 to be perpendicular to the longitudinal axis 32 of the stent 20. The curvature of the connecting members 68 permits the expansion of the stent 20 in a manner similar to the connecting rods 22 of the stent 20.

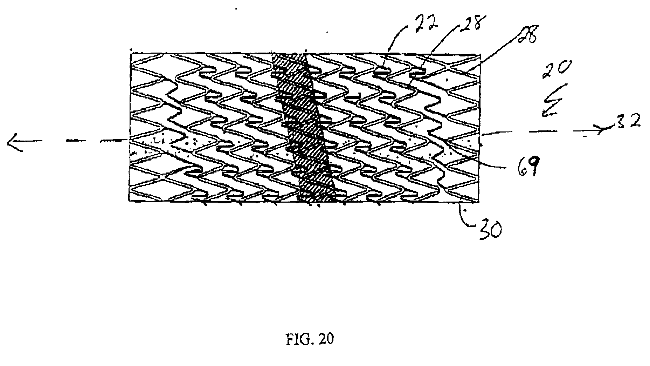

Referring to FIG. 20, in another embodiment of the present invention, the end band 30 is again positioned so that the peaks of the undulations 28 of the end turn of the band 30 are substantially aligned perpendicular to a longitudinal axis 32 of the stent 20. Connecting members 69 are disposed between the end band 30 and the other bands of the undulations 28. The connecting members 69 connect peaks of the undulations 28 of the main pattern of the stent 20 to the undulations 28 of the end turn of the band 30 of the stent 20. The connecting members 68 in the embodiment of FIG. 20 are substantially in a undulation shape with two opposing angle peaks. The connecting member 68 can thus deform with expansion of the stent 20.

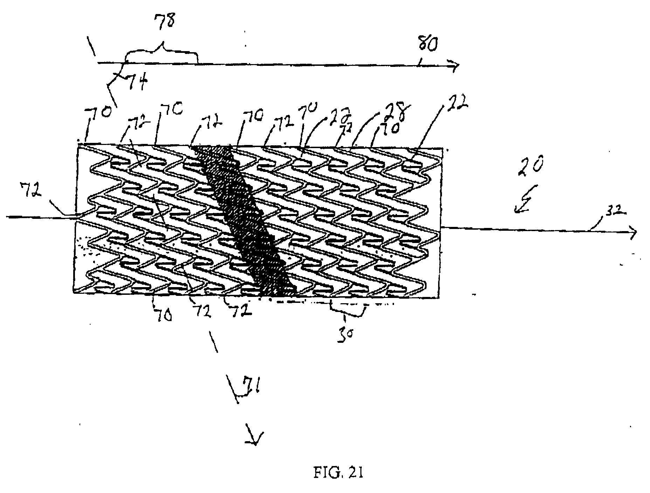

Referring to FIG. 21, in another embodiment of the present invention, the angle of each turn of the band 30 of the undulations 28 is decreased relative to the longitudinal axis 32 of the stent 20, so that a particular turn of the band 30 traces a path about the circumference of the stent 20 that results in a space between successive turns of the band 30 sufficient to dispose another turn in between. Thus, in addition to a first turn 70 that can be traced in the pattern that is laser cut from the tube of the stent 20, a second turn 72 can be traced in positions between each wrap of the first turn 70 of the band 30. In the embodiment of FIG 21, the connecting rods 22 are substantially in the "Z" shape of the connecting members 22 of FIG. 6. The addition of the second turn 72 and the decrease in the angle between a line formed by the peaks of each turn of the band 30 and the longitudinal axis 32 of the stent 20 results in the end of the stent being relatively square compared to a stent that commences at one end and consists only of a single turn. Referring still to FIG. 21, the angle 74 is depicted between the angle of a line 71 formed between the peaks of a band 70, 72 and a line 80 that is parallel to the longitudinal axis 32 of the stent 20. The size of the angle 74 determines that a distance 78 between the beginning of one wrap of the turn 70 of the band 30 and the beginning of the next wrap of the turn 70 of the band 30. The smaller the angle 74, the greater the distance between successive turns of the band 30. The angle 74 can be adjusted to cause the turns to wrap the stent 20 at integral multiples of the width of the band 30, so that other turns can be disposed in the space left by a particular turn. An angle 74 that permits two turns to be disposed adjacent to each other may be described as creating a "double pitch" stent, while an angle that permits three turns to be disposed adjacent to each other may be described as providing a "triple pitch" stent, etc.

It should be noted that the description of the turns 70, 72 is for purposes of identifying the characteristic geometry of the band 30. In fact, the turns 70, 72 are not distinct elements but rather part of an integrated lattice structure that is laser cut from a tube of stent material. Thus, the identification of the turns 70, 72, the connecting members 22, the undulations 28 and the other elements of the stent 20 are for purposes of clarifying the underlying geometry of the lattice or scaffold that makes up the stent 20.

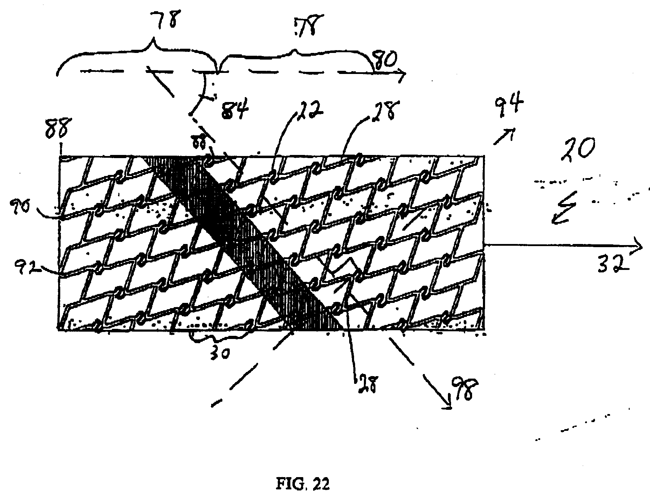

Referring to FIG. 22 in another embodiment of the present invention, a "triple pitch" pattern is provided. The angle 84 between the line 80 that is parallel to the longitudinal axis 32 of the stent 20 is sufficiently small that the distance 78 between the commencement of a first turn 88 and the return of the turn to a similar position after being wrapped three hundred sixty degrees about the circumference of the stent 20 is the equivalent of three widths of an undulation 28. Thus two additional turns 90, 92 can be disposed in the space left between successive wraps of the turn 88. The second turn 90 is disposed at a similar angle 84 and commences at the end of the stent 20. The third turn 92 is also disposed at the angle 84 and also commences at the end of the stent 20. Thus, the stent 20 consists of three turns disposed in a wrapped geometry about the circumference of the stent and connected by the connecting rods 22. The connecting rods 22 of the embodiment of FIG. 22 are in a substantially reverse S-shaped configuration.

The peaks of the undulations 28 of the embodiment of FIG. 22 are aligned differently from other embodiments of the present invention. In particular, a line 94 bisecting a undulation 28 is perpendicular to a line 98 that defines the angle 84 between the turns 88, 90, 92 and the longitudinal axis 32 of the stent 20. Thus, the peaks are aligned perpendicular to the wraps 30, rather than being aligned parallel to the longitudinal axis 32 of the stent 20 as in certain other embodiments of the present invention. The use of three turns 88, 90, 92, with each turn commencing at an end of the stent 20, results in a substantially square end of the stent, without the need for a separate end module. Again, although distinct bands are identified, this identification is for purposes of clarifying the underlying geometry of the stent. In fact, rather than being separate elements, the turns 88, 90, 92 and the connecting members 22 are part of an integrated lattice or scaffold geometry that is laser cut from stent material.

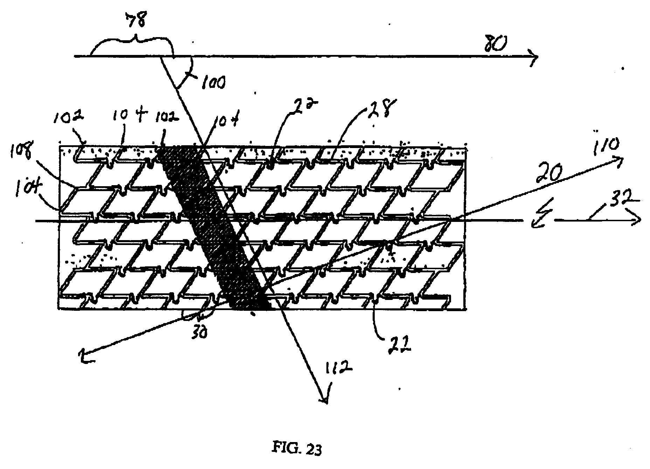

Referring to FIG. 23, in another embodiment of the present invention, another "double pitch" pattern is provided. The angle 100 between the line 80 that is perpendicular to the longitudinal axis 32 of the stent 20, which is defined by the alignment of the peaks of a turn 102 of the undulations 28 is sufficiently small that a particular turn leaves a space of approximately one turn 30 when wrapped about the stent 20 to an angle of three hundred sixty degrees. Thus, a complete wrap of the turn 102 about the length of the stent 20 leaves space for a second turn 104 to be wrapped in the space left by the first turn 102. The second turn 104 commences at the end of the stent and is disposed at a similar angle 100 to the longitudinal axis 32 of the stent 20. Thus, the second turn 104 fits in the space left by the first turn 102. Each turn returns to a position a distance 78 further along the length of the stent 20 with each three hundred sixty degree wrap of the turn. The second turn 104 commences with a connection 108 to the first turn 102, so that the second turn 104 is integrated with the first turn 102. The successive turns 102 and 104 are connected by the connecting rods 22. The connecting members 22 of the embodiment of FIG. 23 are in a substantially "U" shaped configuration. The connecting members 22 perform a similar function to those of other embodiments of the present invention upon expansion of the stent 20. In the embodiment of FIG. 23, the peaks of the undulations 28 are aligned so that each peak is bisected by a line that is parallel to a line 110 that is perpendicular to a line 112 that is defined by the peaks of the undulations 28 of a turn 102, 104. The line 112 is at the angle 100 to the longitudinal axis 32 of the stent 20, and the line 110 is perpendicular to the line 112. Thus, the peaks are substantially aligned perpendicular to the direction of the wraps 30 of the turns 102, 104. As in other embodiments of the present invention, the turns 102, 104, the connecting members 22, the undulations 28, the end module 108 and the other elements of the invention are disclosed for purposes of clarifying the underlying geometry of the stent. In fact, all of these elements are part of an integrated lattice or scaffold structure that is laser cut from a tube.

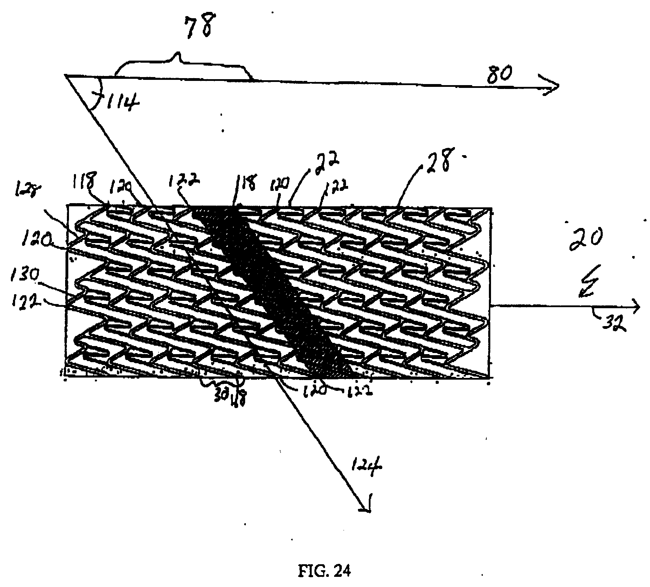

Referring to FIG. 24, in another embodiment of the present invention, another "triple pitch" embodiment is provided. A first turn 118 is disposed in a wrap that is substantially at an angle 114 that is defined by peaks of the undulations 28 of the wrap of the turn 118 and that is sufficiently small that a three hundred sixty degree wrap of the turn 118 results in the turn having moved along the longitudinal axis 32 of the stent to a position that is sufficient for two additional turns 120, 122 to be disposed in the space between successive wraps of the turn 118. The turn 120 commences at the end of the stent 20 and is disposed in the space not filled by the turn 118 at substantially the same angle 114 to the longitudinal axis 32 of the stent. A third turn 122 also commences at the end of the stent 20 and wraps about the stent circumference at substantially the same pitch angle 114. Thus, the turns 120, 122 are disposed in the space left by the first turn 118. Thus, three successive turns 118, 121, 122 wrap the stent from end to end, with peaks of the undulations 28 of each of the turns 118, 121, 122 being disposed along a line 120 that forms an angle 114 with a line 80 that is parallel to the longitudinal axis 32 of the stent 20.

The connecting rods 22 of the embodiment of FIG. 24 are in a substantially "Z" configuration, similar to the connecting members 22 of the embodiments of FIG. 6. The connecting members 22 permit expansion of the stent in a manner similar to the connecting members 22 of other embodiments of the present invention. The turn 120 is commenced at an end of the stent by a special module 128 that connects the turn 120 to the turn 118. The turn 122 is also connected to the turn 120 at the end of a stent 20 by a special module 130. The special modules 128, 130 permit each turn to commence at the end of the stent 20, which causes the end of the stent 20 to have a substantially square end as opposed to an offset end that would be defined by a single, wrapped helical band. Again, the description of the turns 118, 120, 130, the connecting members 22 and the other elements of the stent 20 is for purpose of identifying the underlying geometry of the scaffold or lattice that defines stent. The turns are not in fact distinct elements, but rather part of the underlying geometry that is laser cut from stent material.

A high scaffold stent in accordance with the present invention may be manufactured of stainless steel, such as 316 stainless steel, or other suitable biocompatible material. In embodiments of the present invention it is desirable to provide radiopacity to the stents.

In one embodiment of the invention, any of the different stent configurations can be manufactured by laser cutting the tube from a radiopaque material such as tantalum, gold, platinum, iridium and the like. In other embodiments of the invention it may be desirable to have only part of the stent be made from a radiopaque material; that is, it may be desirable to have the stent ends identifiable by radiopaque markers, while the rest of the stent is relatively more transparent to X-rays, so that the interior of the stent can be seen using various viewing techniques, such as fluoroscopy. One embodiment of a radiopaque marker inserts radiopaque materials into a space, such as the insert spaces of the embodiment depicted in FIG. 6. Gold, platinum, tantalum or other radiopaque materials can be inserted into the spaces that are laser cut into the stent pattern.

In another embodiment of the invention, radiopacity may be provided by plating ends of the stent with radiopaque material. Thus, the stent could be dipped into liquid gold, platinum, or other radiopaque material, then cooled, so that the ends of the stent are coated with the radiopaque material. Alternatively, the ends of the stent may be plated through other conventional plating or coating processes, such as electroplating.

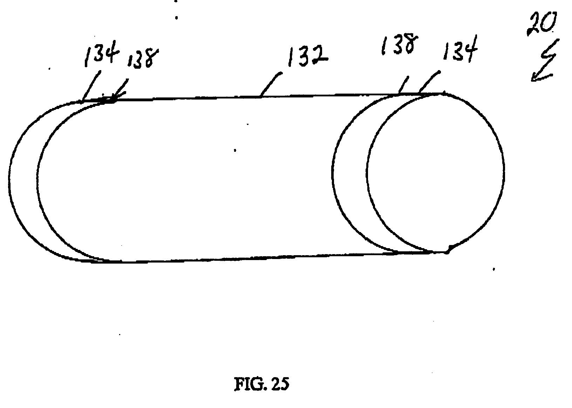

In another embodiment of the invention, depicted in FIG. 25, a stainless steel tube 132 may be extended by providing a pair of end turns 134 that are made of radiopaque material, such as gold, platinum or tantalum. The end turns 134 may be seam welded at a weld 138 at each end of the stainless steel tube 132. Any combination of materials wherein the end turns are more radiopaque than a center turn, or vice versa, is encompassed by the present invention.

## Claims

**1.** A stent, comprising:
undulations having peaks and being disposed in a helical pattern about the circumference of the stent; and
connecting rods connecting a plurality of the peaks.

**2.** The stent of claim 1, wherein a portion of the stent is radiopaque.

**3.** The stent of claim 1, wherein, the stent is formed from a wrapped band of material.

**4.** The stent of claim 1, wherein at least one radiopaque marker is disposed in a portion of the stent.

**5.** The stent of claim 1, wherein at least one end of the stent is electro-plated with a radiopaque material.

**6.** The stent of claim 1, wherein at least one end of the stent is dipped in a radiopaque material.

**7.** The stent of claim 1, wherein the connecting rods are substantially Z-shaped.

**8.** The stent of claim 1, wherein the connecting rods are in a substantially S-shaped geometry.

**9.** The stent of claim 1, wherein the connecting rods are in a substantially J-shaped geometry.

**10.** The stent of claim 1, wherein the connecting rods include a plurality of curved portions and a plurality of substantially straight portions between such curved portions, wherein a first curved portion is oriented oppositely to a second curved portion.

**11.** The stent of claim 1, wherein the connecting rods are disposed between alternate peaks of the undulations.

**12.** The stent of claim 1 and further including a longitudinal axis wherein at least one end of the stent includes undulations that terminate in a plane substantially perpendicular to the longitudinal axis.

**13.** The stent of claim 12, wherein the undulations of the at least one end include progressively different length undulations.

**14.** A stent having a proximal end and a distal end, for supporting a vessel wall, comprising:
a helical pattern of undulations defining the stent, wherein the vessel wall coverage of the stent at the proximal or the distal end is independent of the orientation of the helical pattern about a longitudinal axis of the stent.

**15.** A method of making an expandable prosthesis, comprising:
providing a tube; and
removing a portion of the tube to form undulations disposed in a helical pattern about the tube.

**16.** A method of claim 15 and further comprising:
leaving undulations that terminate in a plane substantially perpendicular to the longitudinal axis at one end of said stent.

**17.** The method of claim 15, further comprising:
removing a portion of the tube to provide connecting rods to connect at least one pair of the undulations.

**18.** The method of claim 15, further comprising:
providing a substantially square end of the stent.

**19.** The method of claim 18, wherein providing a substantially square end includes providing an end module having progressively different length undulations.

**20.** The method of claim 17, wherein the connecting rods are disposed between alternate peaks of the undulations.

**21.** A method of treating vessels in the region of a bifurcation, comprising;
providing a stent having an exterior wall including a helical pattern of undulations having peaks and connecting rods that connect the peaks and that define a space between the peaks and connecting rods;
positioning the stent in a first location in the region of the bifurcation;
expanding the space defined by the connecting rods; and
inserting a dilating means through the space into a second location in the region of the bifurcation,

**22.** The method of claim 21, wherein the connecting rods connect alternate peaks at the undulations.

**23.** The method of claim 21, wherein expanding the space defined by the connecting rods comprises:
inserting a balloon in the space; and
expanding the balloon.

**24.** The method of claim 21, wherein the connecting rods include a plurality of curved portions and a plurality of substantially straight portions between such curved portions, wherein a first curved portion is oriented oppositely to a second curved portion of each connecting rod.

**25.** The method of claim 21, wherein the dilating means is a second, expandable stent.

**26.** A method of treating vessels in the region of a bifurcation, comprising:
providing a stent having a proximal region, a distal region and a central region and an exterior wall including a helical pattern of undulations throughout the central region of the stent, the wall having peaks and connecting rods that connect the peaks and that define a space between peaks and connecting rods;
positioning the stent in a first location in the region of the bifurcation;
expanding the space defined by the connecting rods; and
inserting a dilating means through the space into a second location in the region of the bifurcation.

**26.** A method of claim 25 wherein said dilating means is a second expandable stent.

**27.** A method of claim 25 wherein said stent has a series of connecting rods of varying length disposed between an end module in said proximal region and said central region.

**28.** A method of claim 25 wherein said stent has a series of connecting rods of varying length disposed between an end module in said distal region and said central region.
